# EUROPÄISCHE PATENTANMELDUNG

(11) **EP 4 216 574 A1**
(43) Veröffentlichungstag der Anmeldung: **26.07.2023**
(21) Anmeldenummer: 22215096.3
(22) Anmeldetag: 20.12.2022
(51) Int. Cl.: H04R 25/00

(54) **VERFAHREN FÜR EIN HÖRSYSTEM ZUR ANPASSUNG EINER MEHRZAHL AN SIGNALVERARBEITUNGSPARAMETERN EINES HÖRINSTRUMENTES DES HÖRSYSTEMS**

(30) Priorität: 25.01.2022 DE 102022200810
(71) Anmelder: Sivantos Pte. Ltd., Singapore 539775 (SG)
(72) Erfinder: WIEDENBRÜG, Cornelia, 91058 Erlangen (DE); HANNEMANN, Ronny, 91058 Erlangen (DE); SCHINKEL-BIELEFELD, Nadja, 91058 Erlangen (DE); LUGGER, Marko, 91058 Erlangen (DE)
(74) Vertreter: FDST Patentanwälte

(57) **Zusammenfassung**

Die Erfindung betrifft ein Verfahren für ein Hörsystem (1) zum Anpassen einer Mehrzahl an Signalverarbeitungsparametern (46) eines Hörinstrumentes (2) des Hörsystems (1), gemäß denen wenigstens ein erstes Eingangssignal (16), welches durch einen ersten Eingangswandler (8) des Hörinstrumentes (2) aus einem Umgebungsschall (15) erzeugt wird, zu einem Ausgangssignal (18) verarbeitet wird, wobei verfahrensgemäß anhand des ersten Eingangssignals (16) eine Anzahl an akustischen Indikatoren (IndA) für eine vorliegende temporäre Umgebungssituation (38) eines Benutzers des Hörinstrumentes (2) ermittelt wird, wobei anhand wenigstens eines ersten Sensors (21) des Hörsystems (1) eine Anzahl an peripheren Indikatoren (IndP) für die vorliegende temporäre Umgebungssituation (38) ermittelt wird, wobei anhand der akustischen und peripheren Indikatoren (IndA, IndP) ermittelt wird, ob die vorliegende temporäre Umgebungssituation (38) in eine Klasse (40) aus einer Mehrzahl an bekannten Klassen (40) für temporäre Umgebungssituationen fällt, und, wenn dies nicht der Fall ist,
eine neue Klasse (40a) für temporäre Umgebungssituationen definiert wird, welche mittels des bzw. der jeweils ermittelten akustischen und peripheren Indikatoren (IndA, IndP) charakterisiert ist, und dabei der Mehrzahl an Signalverarbeitungsparametern (46) jeweils Parameterwerte (44a) zugewiesen werden, gemäß denen das erste Eingangssignal (16) bei einem späteren Vorliegen einer temporären Umgebungssituation (38) aus der neuen Klasse (40a) zu einem Ausgangssignal (18) zu verarbeiten ist.

## Beschreibung

Die Erfindung betrifft ein Verfahren für ein Hörsystem zum Anpassen einer Mehrzahl an Signalverarbeitungsparametern eines Hörinstrumentes des Hörsystems, wobei, gemäß denen wenigstens ein erstes Eingangssignal, welches durch einen ersten Eingangswandler des Hörinstrumentes aus einem Umgebungsschall erzeugt wird, zu einem Ausgangssignal verarbeitet wird, welches durch einen Ausgangswandler des Hörinstrumentes in ein Ausgangsschallsignal umgewandelt wird, wobei anhand des ersten Eingangssignals und/oder anhand eines weiteren Eingangssignals, welches durch einen weiteren Eingangswandler des Hörsystems aus dem Umgebungsschall erzeugt wird, eine Anzahl an akustischen Indikatoren für eine vorliegende Umgebungssituation eines Benutzers des Hörinstrumentes ermittelt wird, wobei eine neue Klasse für eine Umgebungssituation definiert wird, und dabei der Mehrzahl an Signalverarbeitungsparametern jeweils eine entsprechende Mehrzahl an Parameterwerten zugewiesen wird, nach deren Maßgabe das erste Eingangssignal bei einem späteren Vorliegen einer Umgebungssituation aus der neuen Klasse zu einem Ausgangssignal zu verarbeiten ist.

In einem Hörgerät, welches für eine Versorgung einer Hörschwäche vorgesehen ist, wird meist durch einen oder mehrere Eingangswandler (wie z.B. einem bzw. mehreren Mikrofonen) aus einem Umgebungsschall eines oder mehrere Eingangssignale erzeugt. Jedes Eingangssignal wird anschließend, insbesondere benutzerspezifisch anhand der audiologischen Vorgaben eines Benutzer des Hörgerätes, verarbeitet, wobei einzelne Signalanteile des oder der Eingangssignale z.B. frequenzbandabhängig verstärkt und/oder komprimiert werden können. Mittels dieser Signalverarbeitung wird ein Ausgangssignal aus dem bzw. den Eingangssignalen erzeugt, und dieses durch einen Ausgangswandler (wie z.B. einen Lautsprecher) in einen Ausgangsschall umgewandelt.

Hierbei ist es oft üblich, dass für den Benutzer des Hörgerätes eine individuelle Anpassung dahingehend erfolgt, welche Parameterwerte einzelner Signalverarbeitungsparameter (wie z.B. Verstärkungsfaktoren, Kompressionsverhältnisse oder - kniepunkte der besagten frequenzbandabhängigen Verstärkung bzw. Kompression, aber auch Parameter zur Unterdrückung von Störgeräuschen oder einer akustischen Rückkopplung) in Abhängigkeit des bzw. der Eingangssignale zugewiesen werden. Einzelne Parameterwerten bestimmter Signalverarbeitungsparameter können dabei insbesondere auch abhängig von sog. Hörsituationen zugewiesen werden. Durch diese Hörsituationen werden einzelne Klassen typischer akustische Umgebungen definiert, sodass bei einem aktuellen Vorliegen einer solchen Hörsituation a priori immer dieselben vordefinierten Parameterwerte (als sog. Hörprogramm) für die Signalverarbeitungsparameter angewandt werden.

Eine Abstimmung dahingehend, wie einzelne Parameterwerte in bestimmten Hörsituationen anzuwenden sind, ist meist von einem Hörgeräteakustiker o.ä. durchzuführen. Dies ist für den Benutzer mit einem Aufwand verbunden (da er den Hörgeräteakustiker aufsuchen muss).

Zunehmend werden auch Hörinstrumente, welche nicht primär für die Versorgung von Hörschwächen vorgesehen sind, wie z.B. Earbuds oder kabellose Ohr-Kopfhörer, dennoch dazu eingesetzt, bis zu einem gewissen Grad eine Unterstützung des Benutzers beim Hören bieten zu können. Gerade für den o.g. Einsatz in Abhängigkeit von Hörsituationen sind diese jedoch primär nicht ausgelegt, und wenn, würde auch hier wohl ein Gang zum Hörgeräteakustiker erforderlich.

Der Erfindung liegt daher die Aufgabe zugrunde, ein Verfahren anzugeben, mittels dessen sich Signalverarbeitungsparameter eines Hörinstruments möglichst einfach und durch den Benutzer selbst anpassen lassen, sodass die Signalverarbeitungsparameter durch die Anpassung für einen umgebungsabhängigen Betrieb individuell auf den Benutzer abgestimmt sind.

Die genannte Aufgabe wird erfindungsgemäß gelöst durch ein Verfahren für ein Hörsystem zum Anpassen einer Mehrzahl an Signalverarbeitungsparametern eines Hörinstrumentes des Hörsystems, gemäß denen wenigstens ein erstes Eingangssignal, welches durch einen ersten, insbesondere elektroakustischen, Eingangswandler des Hörinstrumentes aus einem Umgebungsschall erzeugt wird, zu einem Ausgangssignal verarbeitet wird, welches durch einen, insbesondere elektroakustischen, Ausgangswandler des Hörinstrumentes in ein Ausgangsschallsignal umgewandelt wird.

Hierbei ist vorgesehen, dass anhand des ersten Eingangssignals , eine Anzahl an akustischen Indikatoren für eine vorliegende temporäre Umgebungssituation eines Benutzers des Hörinstrumentes ermittelt wird, anhand wenigstens eines ersten Sensors des Hörsystems eine Anzahl an peripheren Indikatoren für die vorliegende temporäre Umgebungssituation ermittelt wird, und anhand des bzw. der akustischen Indikatoren und anhand des bzw. der peripheren Indikatoren ermittelt wird, ob die vorliegende temporäre Umgebungssituation in eine Klasse aus einer Mehrzahl an bekannten Klassen für temporäre Umgebungssituationen fällt.

Weiter ist vorgesehen, dass im Fall, dass die vorliegende temporäre Umgebungssituation in keine der bekannten Klassen für temporäre Umgebungssituationen fällt, eine neue Klasse für eine temporäre Umgebungssituation definiert wird, welche mittels des bzw. der jeweils ermittelten akustischen und peripheren Indikatoren charakterisiert ist, und dabei der Mehrzahl an Signalverarbeitungsparametern jeweils eine entsprechende Mehrzahl an Parameterwerten zugewiesen wird, nach deren Maßgabe das erste Eingangssignal bei einem späteren Vorliegen einer temporären Umgebungssituation aus der neuen Klasse zu einem Ausgangssignal zu verarbeiten ist. Vorteilhafte und teils für sich gesehen erfinderische Ausgestaltungen sind Gegenstand der Unteransprüche und der nachfolgenden Beschreibung.

Das Verfahren stellt mit anderen Worten eine Möglichkeit bereit, eine Mehrzahl an Signalverarbeitungsparametern eines Hörinstruments anzupassen, welche im laufenden Betrieb des Hörinstruments dazu verwendet werden, wenigstens ein in beschriebener Weise erzeugtes erstes Eingangssignal des Hörsystems zu einem in beschriebener Weise wiederzugebenden Ausgangssignal zu verarbeiten. Das Verfahren wird dabei mittels eines Hörsystems durchgeführt. Das Hörsystem umfasst dabei zumindest auch das Hörinstrument, und kann ggf. noch weitere Geräte umfassen, insbesondere ein mit dem Hörinstrument verbindbares Hilfsgerät.

Hierbei werden zunächst akustische Indikatoren und periphere, also insbesondere nicht-akustische Indikatoren ermittelt, welche Aufschluss über eine vorliegende temporäre Umgebungssituation des Benutzers liefern. Eine solche temporäre Umgebungssituation ist dabei, zu verstehen als eine Situation im Alltag des Benutzers, welche nicht nur anhand von akustischen Merkmalen (wie im Fall der Hörsituation) charakterisiert ist, sondern auch durch weitere Merkmale, z.B. bewegungs- und/oder ortsbezogene Merkmale.

Dabei wird zunächst anhand des ersten Eingangssignals, und ggf. eines weiteren Eingangssignals des Hörsystems eine Anzahl an akustischen Indikatoren ermittelt. Das weitere Eingangssignal kann dabei durch einen weiteren Eingangswandler des Hörinstruments erzeugt werden, etwa bei einem für Richtmikrofonie eingerichteten monauralen Hörgerät oder auch einem binauralen Hörgerät als Hörinstrument. Der bzw. die akustischen Indikatoren werden dabei bevorzugt anhand einer spektralen Analyse des ersten Eingangssignals ermittelt, und sind insbesondere derart ausgestaltet, dass sie einen Rückschluss auf eine sog. Hörsituation erlauben, welche im Moment der Aufnahme des ersten Eingangssignals vorliegt.

Als akustische Indikatoren werden dabei bevorzugt direkt eine Hörsituation, und/oder wenigstens eine Kenngröße für einen Sprachanteil und/oder eine eigene Sprachaktivität des Benutzers im vom ersten Eingangswandler aufgenommenen Umgebungsschall, und/oder eine Kenngröße für einen Rauschanteil, insbesondere ein Windrauschen, im Umgebungsschall, und/oder eine Kenngröße für tonale Signale im Umgebungsschall und/oder eine Kenngröße für eine Direktionalität (insbesondere deren Stärke und genaue Richtung) im Umgebungsschall ermittelt. Ferner können als akustische Indikatoren auch Kenngrößen bzgl. der Onsets und/oder der Rhythmik eines Sprachsignals im Umgebungsschall, bzgl. einer Stationarität, und/oder bzgl. eines spektralen Schwerpunkts des Umgebungsschalls verwendet werden. Anhand der genannten Kenngrößen lässt sich auch auf eine Hörsituation schließen. Im bereits angesprochenen Fall eines Hörinstruments mit einem weiteren Eingangswandler, welches zur Richtmikrofonie eingerichtet ist, kann auch eine Kenngröße für eine Direktionalität des Umgebungsschalls als akustischer Indikator ermittelt werden.

Als periphere Indikatoren werden dabei bevorzugt eine kardiovaskuläre Kenngröße (insbesondere ein Blutdruck und/oder ein Pulsschlag und/oder eine Herzfrequenz des Benutzers), und/oder ein Bewegungszustand des Benutzers, und/oder eine Ortsposition des Benutzers und/oder eine Körpertemperatur des Benutzers, und/ und/oder eine Kenngröße für ein Stressempfinden des Benutzers ermittelt. Insbesondere kann auch ein Blutzuckerspiegel als peripherer Indikator ermittelt werden. Als erster Sensor zum Ermitteln des bzw. der peripheren Indikatoren wird dabei im Fall der Herzfrequenz bzw. des Pulsschlags bzw. des Blutdrucks bevorzugt ein Photoplethymographie-Sensor (PPG-Sensor), im Fall des Bewegungszustandes bevorzugt ein Beschleunigungssensor, im Fall der Ortsposition bevorzugt ein GPS-Empfänger und/oder eine über eine WLAN-Verbindung bezogene Ortsinformation und im Fall der Körpertemperatur bevorzugt ein Thermometer verwendet. Weiter kann als erster Sensor ein Bioimpedanzsensor ein Sensor einer elektrodermalen Aktivität, ein Altimeter und/oder ein Helligkeitssensor verwendet und anhand des jeweiligen Sensorsignals ein entsprechender peripherer Indikator erzeugt werden.

Die peripheren Indikatoren erlauben somit eine feinere Zuordnung der vorliegenden temporären Umgebungssituation, als dies allein mittels des bzw. der akustischen Indikatoren möglich wäre. So kann z.B. eine laute Umgebung mit vielen Gesprächsanteilen, viel Hall und weiter vielen Störgeräuschen einerseits gegeben sein durch ein Gespräch in einem Restaurant o.ä. (sog. "Cocktail-Party"-Hörsituation), oder auch durch die Teilnahme an einem Mannschaftssport in einer Halle.

Eine Abgrenzung hierbei lässt sich anhand der peripheren Indikatoren "Bewegungszustand" und ggf. Blutdruck/Puls/Herzfrequenz deutlich effektiver vornehmen als allein anhand von akustischen Indikatoren, für welche die beiden Situationen ggf. schwerer zu unterscheiden sind.

Der erste Sensor kann dabei einerseits im Hörinstrument angeordnet sein, oder andererseits in einem mit dem Hörinstrument verbindbaren, zum Hörsystem gehörenden Hilfsgerät wie z.B. einem Smartphone, einer Smartwatch oder einem Fitnessarmband o.ä. Insbesondere können die akustischen Indikatoren zusätzlich auch anhand eines weiteren Eingangssignals, welches durch einen weiteren elektroakustischen Eingangswandler des Hörsystems (bspw. im Hilfsgerät) aus dem Umgebungsschall erzeugt wird, ermittelt werden.

Die zu einem konkreten Zeitpunkt vorliegende temporäre Umgebungssituation ist somit im Sinne des Verfahrens insbesondere durch die für diesen Zeitpunkt ermittelten akustischen und peripheren Indikatoren charakterisiert. Insbesondere sind dabei Ähnlichkeiten und/oder Unterschiede zu anderen temporären Umgebungssituationen, sowie Klassifizierungen zu einzelnen Klassen, bezogen auf die besagten akustischen und peripheren Indikatoren.

Es wird nun anhand des bzw. der jeweils ermittelten akustischen und peripheren Indikatoren ermittelt, ob die zu einem Zeitpunkt vorliegende temporäre Umgebungssituation in eine Klasse aus einer Mehrzahl an bekannten Klassen für temporäre Umgebungssituationen fällt. Eine derartige Klasse ist dabei bevorzugt dadurch charakterisiert, dass sie für einzelne akustische und periphere Indikatoren, wenn diese ihrer Natur nach kontinuierliche Werte annehmen können (wie z.B. Signal-zu-Rausch-Verhältnis, prozentualer Sprachanteil, Blutdruck, Puls- /Herzfrequenz etc.), Intervalle als Wertebereiche aufweist, sodass eine temporäre Umgebungssituation nur dann in einer Klasse liegen kann, wenn zumindest die kontinuierlichen ihrer akustischen und peripheren Indikatoren in den für die Klasse jeweils entsprechend definierten Intervallen liegen.

Alternativ dazu, oder auch für einzelne der Indikatoren, können Referenzwerte und Abstandsrelationen definiert werden, d.h., für eine Klasse werden jeweils einzelne Referenzwerte konkreter Indikatoren festgelegt, und zusätzlich im durch die jeweiligen Indikatoren definierten Parameterraum Abstandsrelationen bzgl. der Referenzwerte oder auch für Vektoren der Indikatoren bzgl. eines entsprechenden Vektors mit den Referenzwerten definiert. Für nicht-kontinuierliche Indikatoren können sich dabei vergleichbare Abstandsmaße definieren lassen.

Eine Klasse für eine temporäre Umgebungssituationen ist somit gegeben durch eine typische Situation im Alltag des Benutzers, sodass die charakterisierenden Merkmale dieser Situation, bis auf kleine Abweichungen (repräsentiert z.B. durch die o.g. Intervalle), ein Vorliegen der besagten Situation (und dadurch insbesondere auch eine Abgrenzung zu anderen typischen Situationen bzw. Klassen) erkennen lassen. Die vorliegende Tagesumlauf-Umgebungssituation fällt somit insbesondere dann in eine der bekannten Klassen, wenn die vorliegend ermittelten akustischen und peripheren Indikatoren in die für die bekannte Klasse entsprechend definierten Intervalle der zugehörigen Indikatoren fallen, und/oder sonstige Abstandskriterien bezüglich für die Klasse vordefinierter Werte erfüllen.

Einer bekannten Klasse an temporären -Umgebungssituationen ist dabei bevorzugt eine Mehrzahl an Parameterwerten für die entsprechenden Signalverarbeitungsparameter zugewiesen, d.h., bevorzugt wird bei im Fall, dass die vorliegende temporäre Umgebungssituation in eine der bekannten Klassen fällt, die Signalverarbeitung wenigstens des ersten Eingangssignals (und ggf. weiterer Eingangssignale) zum Ausgangssignal gemäß der für die Klasse festgelegten Parameterwerte durchgeführt.

Fällt dabei die vorliegende temporäre Umgebungssituation in keine der bekannten Klassen, so wird anhand des bzw. der vorliegend ermittelten akustischen Indikatoren und anhand des bzw. der vorliegend ermittelten peripheren Indikatoren eine neue Klasse definiert, sodass die neu definierte Klasse durch die besagten Indikatoren charakterisiert ist. Bevorzugt werden dabei auch Abstands- und/oder Ähnlichkeitsrelationen der akustischen und peripheren Indikatoren festgelegt, sodass eine später vorliegende temporäre Umgebungssituation auch dann noch in die neu definierte Klasse fällt, wenn für wenigstens einen der Indikatoren eine Abweichung zum entsprechenden, für die Definition der Klasse verwendeten Wert des Indikators vorliegt, aber diese Abweichung bezogen auf den gesamten Wertebereich und/oder auf die Relation zu anderen Klassen vernachlässigt werden kann.

Bei der Definition der neuen Klasse wird nun der Mehrzahl an Signalverarbeitungsparametern eine entsprechende Mehrzahl an Parameterwerten zugewiesen, nach deren Maßgabe das erste Eingangssignal (und ggf. weitere) zum Ausgangssignal zu verarbeiten ist, wenn zu einem späteren Zeitpunkt eine temporäre Umgebungssituation vorliegt, welche anhand ihrer akustischen und peripheren Indikatoren in die neu definierte Klasse fällt (also wenn die genannten Indikatoren hinreichende Ähnlichkeit mit denjenigen Werten der entsprechenden Indikatoren aufweisen, welche für die Definition der Klass verwendet wurden.

Das beschriebene Vorgehen erlaubt es dem Benutzer bei einem Erkennen einer neuen temporären Umgebungssituation durch das Hörsystem, welche in keine bekannte Klasse fällt, eine neue solche Klasse für eine spätere Verwendung zu definieren. Somit kann der Benutzer, individuell zugeschnitten auf seinen Alltag und seinen Tagesablauf, einzelne Klassen definieren und entsprechende Parameterwerte für die Signalverarbeitungsparameter zuweisen lassen, ohne dass er dafür einen Hörgeräteakustiker o.ä. aufsuchen muss.

Bevorzugt wird dabei für die Definition der neuen Klasse durch eine Bedieneinrichtung des Hörsystems, bspw. über Bedienelemente am Hörinstrument und/oder an bereits erwähntem Hilfsgerät (vorzugsweise über einen Touchscreen wie z.B. bei einem Smartphone), eine erste Bestätigungsanfrage an den Benutzer gerichtet, und die Definition im Fall einer Bestätigung der Anfrage vorgenommen. Es kann dann, wenn die vorliegende temporäre Umgebungssituation gemäß ihrer akustischen und peripheren Indikatoren als mit keiner der bekannten Klassen kompatibel erkannt wird, die Anfrage eingeblendet werden, ob der Benutzer sich gerade in einer neuartigen Situation befindet, und entsprechend eine neue Klasse definiert werden soll. Bestätigt der Benutzer diese Anfrage, wird die Klasse definiert. Falls nicht, unterbleibt die Definition.

Auch wenn die Definition der neuen Klasse auch automatisch erfolgen kann und eine Interaktion mit dem Benutzer hierfür nicht zwingend erforderlich ist, so liefert eine solche Interaktion in der hier beschriebenen Form der Bestätigungsanfrage den Vorteil, besser auf die individuellen Bedürfnisse des Benutzers einzugehen. So ist z.B. nicht alles, was automatisch als eine eigenständige, neue Klasse erkannt würde, für den Benutzer auch tatsächlich von Belang.

Günstigerweise wird bei der Definition der neuen Klasse für die Zuweisung der Mehrzahl an Parameterwerten ein Ähnlichkeitswert der neuen Klasse mit wenigstens einer bestehenden Klasse ermittelt, und die Mehrzahl an Parameterwerten für die neue Klasse anhand des Ähnlichkeitswertes und anhand der Mehrzahl an Parameterwerten der wenigstens einen bestehenden Klasse zugewiesen. Der Ähnlichkeitswert kann z.B. anhand einer Abstandsrelation eines Vektors der Werte der einzelnen vorliegend ermittelten akustischen und peripheren Indikatoren zu einem entsprechenden Vektor von Indikatoren der besagten bestehenden Klasse bzw. Klassen gebildet werden.

Bspw. können in einem Vektorraum, welcher von einzelnen Indikatoren aufgespannt wird, für den Vektor der vorliegend ermittelten Indikatorwerte die "nächstgelegenen" Klassen anhand entsprechender Vektoren (deren Einträge z.B. jeweils durch Referenzwerte für die jeweilige Klasse gegeben sein können) über eine Abstandsrelation im Vektorraum bestimmt werden, und anhand der Abstandsrelation auch der besagte Ähnlichkeitswert (z.B. über eine vorgegebene Skala, welche einem geringeren Abstand gemäß der Abstandsrelation einen höheren Ähnlichkeitswert zuweist). Die Mehrzahl an Parameterwerten kann dann jeweils den Signalverarbeitungsparametern zugewiesen werden, indem die entsprechenden Parameterwerte der nächstgelegenen Klasse bzw. Klassen, jeweils gewichtet anhand des Ähnlichkeitsmaßes, für die Zuweisung herangezogen werden.

Zweckmäßigerweise werden bei der Definition der neuen Klasse für die Zuweisung der Mehrzahl an Parameterwerten mittels einer Kommunikationseinrichtung des Hörsystems der bzw. die ermittelten akustischen und peripheren Indikatoren an eine vom Hörsystem getrenntes Rechensystem mit einer Datenbank übermittelt (insbesondere an eine zentrale Recheneinrichtung mit einer zentralen oder dezentralen Datenbank, wie z.B. einen Cloud-Server), in welcher eine Vielzahl an Klassendefinitionen hinterlegt ist, wobei die Mehrzahl an Parameterwerten für die neue Klasse durch das Rechensystem anhand des bzw. der ermittelten akustischen und peripheren Indikatoren sowie anhand der Klassendefinitionen zugewiesen wird. Der Zugriff auf Klassendefinitionen, welche z.B. für andere Benutzer (von diesen oder auch durch professionelle Hörgeräteakustiker) vorgenommen wurden, und die zugehörigen Indikatoren, erleichtert die Neudefinition von Klassen. Insbesondere erfolgt dabei die Zuweisung im Rechensystem mittels einer künstlichen Intelligenz wie z.B. eines künstlichen neuronalen Netzes o.ä., wodurch die Neudefinition weiter vereinfacht werden kann. Die einzelnen hinterlegten Klassendefinitionen umfassen hierbei bevorzugt auch die entsprechenden Parameterwerte.

Günstigerweise werden hierbei zusätzlich audiologische und/oder biometrische Daten des Benutzers an das Rechensystem übermittelt, wobei in der Datenbank weiter zur jeder Klassendefinition zugehörige audiologische und/oder biometrische Daten einer der jeweiligen Klassendefinition zugeordneten Person hinterlegt sind, und wobei die Mehrzahl an Parameterwerten für die neue Klasse durch das Rechensystem zusätzlich anhand der audiologischen und/oder biometrischen Daten des Benutzers sowie anhand der audiologischen und/oder biometrischen Daten der den jeweiligen Klassendefinitionen zugeordneten Personen zugewiesen wird.

Dies bedeutet insbesondere, dass für die Klassendefinition zusätzlich zu den jeweiligen Parameterwerten, welche den anhand der akustischen und peripheren Indikatoren definierten, hinterlegten Klassen zugewiesen sind, zusätzlich auch audiometrische Daten (wie z.B. Audiogramme) und/oder biometrische Daten (z.B. hinsichtlich Alter, Geschlecht, ggf. Beruf, chronischer Erkrankungen etc.) derjenigen Personen, für welche die Klassen jeweils definiert wurden, erfasst und hinterlegt werden. Die Definition der neuen Klasse zu den vorliegenden Indikatoren kann dann zusätzlich anhand eines Abgleiches der audiologischen bzw. biometrischen Daten erfolgen, sodass bevorzugt diejenigen hinterlegten Klassendefinitionen stärker berücksichtigt werden, für welche die audiologischen bzw. biometrischen der betreffenden Person eine höhere Ähnlichkeit zu denen des Benutzers des Hörinstruments aufweist.

Als weiter vorteilhaft erweist es sich, wenn bei der Definition der neuen Klasse für die Zuweisung der Mehrzahl an Parameterwerten jeweils vorläufige Parameterwerte zugewiesen werden, und das erste Eingangssignal nach Maßgabe der vorläufigen Parameterwerte für die Mehrzahl an Signalverarbeitungsparametern zum Ausgangssignal verarbeitet wird, wobei das dem so erzeugten Ausgangssignal entsprechende Ausgangsschallsignal einem Gehör des Benutzers zugeführt wird, und wobei eine zweite Bestätigungsanfrage an den Benutzer gerichtet wird, und im Fall einer entsprechenden Bestätigung die vorläufigen Parameter als Mehrzahl an Parameterwerten für die Definition der Klasse festgelegt wird. Dies umfasst insbesondere, dass also eine Klassendefinition für die temporäre Umgebungssituation mit den zugehörigen Parameterwerten in einer Art Testbetrieb dem Benutzer vorgeführt wird, und dieser über die zweite Bestätigungsanfrage entscheiden kann, ob er diese vorläufigen Parameterwerte, welche im Testbetrieb verwendet werden, beibehalten möchte.

Bevorzugt wird dabei im Fall einer Ablehnung der zweiten Bestätigungsanfrage eine Anfrage hinsichtlich einer Änderungen eines Parameterwertes für einen bestimmten der Signalverarbeitungsparameter an den Benutzer gerichtet, wobei anhand einer entsprechenden Eingabe des Benutzers eine Änderung am besagten Parameterwert für den bestimmten Signalverarbeitungsparameter vorgenommen wird. Dies bedeutet insbesondere, das im Fall einer Ablehnung der zweiten Bestätigungsanfrage - also wenn der Benutzer den beschriebenen Testbetrieb der neuen Klassendefinition mit den entsprechenden vorläufigen Parametern negativ bewertet - der Benutzer di Möglichkeit erhält, einzelne Signalverarbeitungsparameter gezielt selbst anzupassen. Hierbei kann z.B. die Möglichkeit eröffnet werden, entsprechende Signalverarbeitungsparameter für eine Anpassung auszuwählen, und dann entweder durch zu bestätigende Vorschläge für Parameterwerte oder direkt über eine Art Reglerfunktion einer Benutzerschnittstelle (z.B. einer App) einzustellen, mittels derer die jeweilige Bestätigungsanfrage an den Benutzer ausgegeben wird. Nach Abschluss des Einstellens der Parameterwerte für die gewünschten Signalverarbeitungsparameter kann dann die neue Definition der Klasse z.B. gespeichert werden.

Die Erfindung nennt weiter ein Hörsystem, umfassend ein Hörinstrument mit wenigstens einem Eingangswandler, einer Signalverarbeitungseinrichtung und einem Ausgangswandler, einen ersten Sensor. Dabei ist der Eingangswandler dazu eingerichtet, aus einem Umgebungsschall ein Eingangssignal zu erzeugen, wobei die Signalverarbeitungseinrichtung dazu eingerichtet ist, das Eingangssignal nach Maßgabe einer Mehrzahl an Signalverarbeitungsparametern zu einem Ausgangsignal zu verarbeiten, wobei der Ausgangswandler dazu eingerichtet ist, das Ausgangssignal in ein Ausgangsschallsignal umzuwandeln, wobei der erste Sensor dazu eingerichtet ist, eine Anzahl an peripheren Indikatoren für eine vorliegende temporäre Umgebungssituation eines Benutzers des Hörinstruments zu ermitteln, und wobei das Hörsystem dazu eingerichtet ist, die besagte Mehrzahl an Signalverarbeitungsparametern gemäß dem vorbeschriebenen Verfahren anzupassen.

Das erfindungsgemäße Hörsystem teilt die Vorzüge des erfindungsgemäßen Verfahrens. Die für das Verfahren und für seine Weiterbildungen angegebenen Vorteile können sinngemäß auf das Hörsystem übertragen werden.

Nachfolgend wird ein Ausführungsbeispiel der Erfindung anhand von Zeichnungen näher erläutert. Hierbei zeigen jeweils schematisch:
- Fig. 1: in einem Blockschaltbild ein Hörsystem mit einem Hörgerät und einem Smartphone, welches dazu eingerichtet ist, eine allgemeine Situation im Tagesablauf eines Benutzers zu erfassen,
- Fig. 2: in einem Blockdiagramm den Ablauf eines Verfahrens, mittels des Hörsystems nach Fig. 1 für das Hörgerät abhängig von der erfassten Situation Signalverarbeitungsparameter festzulegen.

Einander entsprechende Teile und Größen sind in allen Figuren jeweils mit denselben Bezugszeichen versehen.

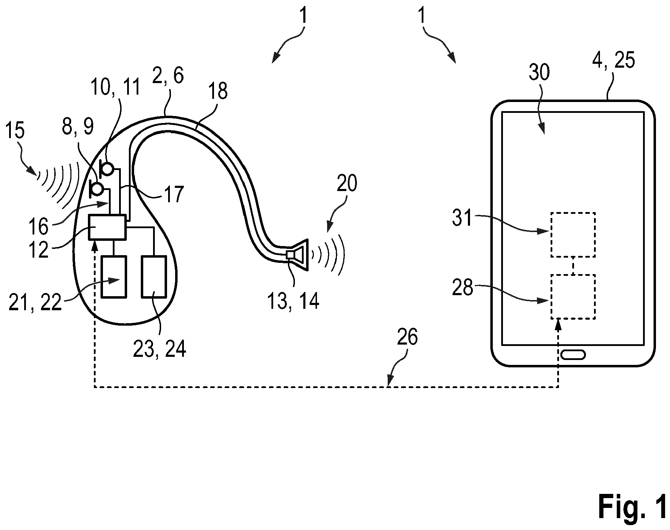

In Figur 1 ist schematisch in einem Blockschaltbild ein Hörsystem 1 dargestellt, welches ein Hörinstrument 2 und ein Hilfsgerät 4 umfasst. Das Hörinstrument 2 ist vorliegend ausgestaltet als ein Hörgerät 6, und weist entsprechend einen ersten elektroakustischen Eingangswandler 8, einen zweiten elektroakustischen Eingangswandler 9, eine mit beiden besagten Eingangswandlern 8, 10 verbundene Signalverarbeitungseinrichtung 12 sowie einen mit der Signalverarbeitungseinrichtung 12 verbundenen elektroakustischen Ausgangswandler 14 auf. Der erste und der zweite elektroakustische Eingangswandler sind dabei gegeben durch ein erstes Mikrofon 9 bzw. ein zweites Mikrofon 11, der elektroakustischen Ausgangswandler 14 durch einen Lautsprecher 13. Das erste und das zweite Mikrofon 9, 11 sind jeweils dazu eingerichtet, aus einem Umgebungsschall 15 ein erstes Eingangssignal 16 bzw. ein zweites Eingangssignal 17 zu erzeugen. Das erste Eingangssignal 16 und das zweite Eingangssignal 17 werden in der Signalverarbeitungseinrichtung 12 zu einem Ausgangssignal 18 verarbeitet, welches durch den Lautsprecher 13 in ein Ausgangsschallsignal 20 umgewandelt wird. Bei der Verarbeitung der beiden Eingangssignale 16, 17 in der Signalverarbeitungseinrichtung 12 kann insbesondere ein Hörverlust eines nicht näher dargestellten Benutzers des Hörgerätes 6 dahingehend berücksichtigt werden, dass durch eine frequenzbandabhängige Verstärkung und/oder Kompression der Eingangssignale 16, 17 der Hörverlust zumindest teilweise kompensiert wird. Hierfür werden in der Signalverarbeitungseinrichtung 12 einzelne Signalverarbeitungsparameter für die Verarbeitung des ersten und des zweiten Eingangssignals 16, 17 in einer noch zu beschreibenden Abhängigkeit von äußeren Gegebenheiten auf entsprechende Parameterwerte eingestellt.

Weiter weist das Hörgerät 6 als einen ersten Sensor 21 einen PPG-Sensor 22 auf, welcher ebenfalls mit der Signalverarbeitungseinrichtung 12 verbunden ist. Der PPG-Sensor 22 ist insbesondere dazu eingerichtet, eine Herzfrequenz und/oder einen Blutdruck des Benutzers zu messen. Das Hörgerät 6 weist überdies im vorliegenden Ausführungsbeispiel als einen weiteren Sensor 23 einen Beschleunigungssensor 24 auf, welcher ebenfalls mit der Signalverarbeitungseinrichtung 12 verbunden ist, und mittels dessen eine Bewegungsänderung des Benutzers des Hörgerätes 6 erfasst werden kann. Durch eine Integration der Beschleunigung in der Signalverarbeitungseinrichtung 12 kann somit auch eine Bewegung des Benutzers erfasst werden.

In noch zu beschreibender Weise werden die besagten Signalverarbeitungsparameter für die Verarbeitung der beiden Eingangssignale 16, 17 in Abhängigkeit von äußeren Gegebenheiten auf entsprechende Parameterwerte eingestellt. Insbesondere soll dabei dem Benutzer ermöglicht werden, derartige Abhängigkeiten von den äußeren Gegebenheiten, also einen Zusammenhang zwischen den Gegebenheiten und der entsprechenden Zuweisung von Parameterwerten für die Signalverarbeitungsparameter, selbst gemäß seinen individuellen Bedürfnissen festzulegen. Hierzu wird insbesondere das vorliegend als ein Smartphone 25 ausgestaltete Hilfsgerät 4 verwendet, welches über eine Bluetooth-Verbindung 26 mit dem Hörgerät 6 verbindbar ist. Dazu weisen das Hörgerät 6 und das Smartphone 25 jeweils entsprechend eingerichtete, in Figur 1 nicht näher dargestellte Sende- und Empfangsvorrichtungen (zum Beispiel Antennen) zum Aufbau besagter Bluetooth-Verbindung 26 auf. Das Smartphone 25 weist zudem für nachfolgend beschriebene Verarbeitungsschritte eine Prozessoreinheit 28 auf, sowie einen Touchscreen 30, mittels dessen über eine entsprechende Applikation Benutzereingaben durch den Träger des Hörgerätes 6 bzw. des Systems 1 vorgenommen werden können. Überdies weist das Smartphone 25 einen mit der Prozessoreinheit 28 verbundenen, als einen für das Erfassen der äußeren Gegebenheiten als weiteren Sensor zu verwendenden GPS-Sensor 31 auf.

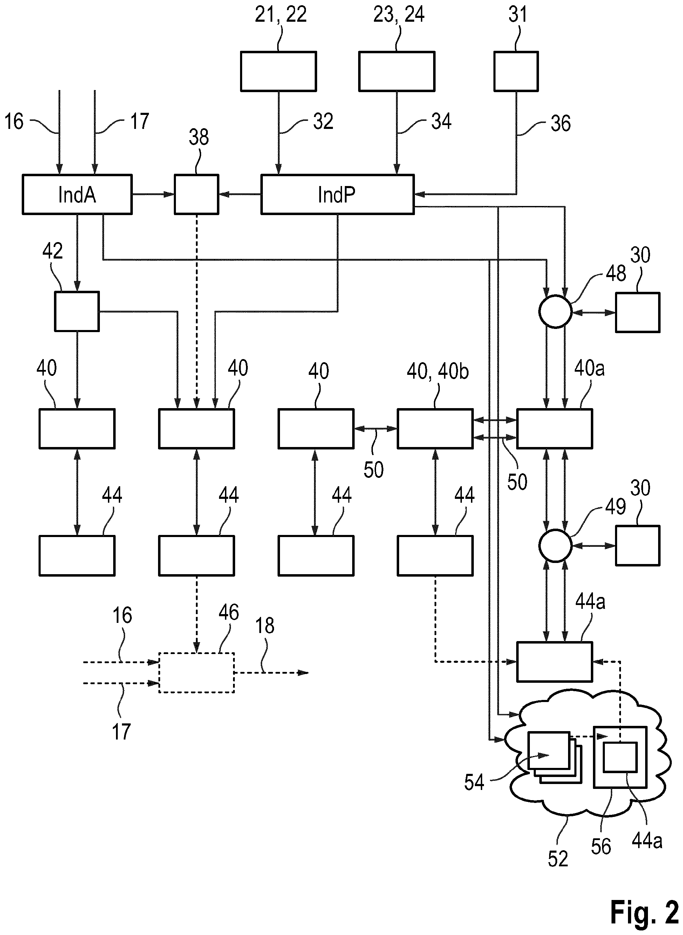

In Figur 2 ist schematisch in einem Blockdiagramm der Ablauf eines Verfahrens dargestellt, mittels dessen den Signalverarbeitungsparametern des Hörgerätes 6 nach Figur 1 in Abhängigkeit von äußeren Gegebenheiten einzelne Parameterwerte zugewiesen werden. Anhand des ersten Eingangssignals 16 und des zweiten Eingangssignals 17 werden zunächst durch eine Analyse besagter Signale in der Signalverarbeitungseinheit 12 akustische Indikatoren IndA gewonnen, welche Aufschluss über die äußeren Gegebenheiten wie Hörsituation oder auch Umgebung im Allgemeinen erlauben. Hierfür werden die beiden Eingangssignale 16, 17 bevorzugt in einzelne Frequenzbänder zerlegt. Die akustischen Indikatoren können dabei bevorzugt direkt eine Hörsituation umfassen, oder auch eine Kenngröße für einen Sprachanteil, für eine eigene Sprachaktivität des Benutzers, für einen Rauschanteil oder für tonale Signale im Umgebungsschall. Weiter wird bevorzugt auch die Direktionalität von Schallsignalen einzelner Schallquellen im Umgebungsschall mittels Richtmikrofonie (durch eine entsprechende Verarbeitung der beiden Eingangssignale 16, 17) herangezogen, was insbesondere für eine Erkennung der vorliegenden Hörsituation vorteilhaft ist.

Des Weiteren werden nun anhand des ersten Sensors 21 und des weiteren Sensors 23 auch periphere Indikatoren IndP erfasst. Der als PPG-Sensor 22 ausgestaltete erste Sensor 21 erfasst hierbei als periphere Indikatoren IndP wenigstens eine kardiovaskuläre Kenngröße 32 des Benutzers, also z.B. einen Blutdruck und/oder eine Pulsfrequenz. Der als Beschleunigungssensor 24 ausgestaltete weitere Sensor 23 erfasst als einen weiteren peripheren Indikator IndP Bewegungsänderungen des Benutzers, wodurch insbesondere Rückschlüsse auf körperliche Aktivität 34 wie bspw. sportliche Betätigung oder auch körperliche Arbeit möglich sind. Der entsprechende periphere Indikator IndP kann hier auch durch eine detailliertere Auswertung der erfassten Bewegungsänderungen hinsichtlich der genauen Art der körperlichen Aktivität (s.o.) gegeben sein. Zusätzlich kann durch den GPS-Sensor 31 des Smartphones 25 als ein weiterer peripherer Indikator IndP eine Position 36 des Benutzers bezogen werden.

Die akustischen Indikatoren IndA und die peripheren Indikatoren IndP legen dabei eine temporäre Umgebungssituation 38 fest. Diese temporäre Umgebungssituation 38 stellt insbesondere eine Charakterisierung der äußeren Gegebenheiten gemäß der Gesamtheit ihrer erfassten Merkmale dar. Anhand der akustischen Indikatoren IndA und der peripheren Indikatoren IndP wird nun geprüft, ob die vorliegende temporäre Umgebungssituation 38 in eine vorab definierte Klasse 40 für temporäre Umgebungssituationen fällt. Derartige Klassen 40 für temporäre Umgebungssituationen können dabei insbesondere verstanden werden als eine Erweiterung des Konzeptes der Hörsituation auf nicht-akustische Merkmale (erfasst vorliegend anhand der peripheren Indikatoren IndP) für eine weitere Unterscheidung.

Insbesondere kann die Überprüfung, ob die vorliegende temporäre Umgebungssituation 38 in eine der besagten Klassen 40 fällt, auch kaskadiert erfolgen, indem z.B. zunächst anhand der akustischen Indikatoren IndA eine reine Hörsituation 42 ermittelt wird, welche jedoch durch mehrere der Klassen 40 für temporäre Umgebungssituationen gegeben sein kann, und anschließend anhand der peripheren Indikatoren IndP überprüft wird, ob die vorliegende temporäre Umgebungssituation 38 einer der betreffenden Klassen 40 für die vorliegende Hörsituation 42 zugeordnet werden kann.

Jede einzelne der Klassen 40 für temporäre Umgebungssituationen ist dabei einerseits anhand der betreffenden akustischen und peripheren Indikatoren IndA, IndP charakterisiert, wobei bevorzugt auch ein Abstands- und/oder Ähnlichkeitsmaß definiert ist, sodass für zu einem gegebenen Zeitpunkt vorliegende akustische und periphere Indikatoren IndA, IndP anhand des Abstands- bzw. Ähnlichkeitsmaßes bzgl. der entsprechenden Indikatoren der vordefinierten Klassen 40 ermittelt werden kann, in welche Klasse 40 die entsprechend zum Zeitpunkt vorliegende temporäre Umgebungssituation 38 fällt. Ein derartiges Abstands- bzw. Ähnlichkeitsmaß kann für kontinuierliche Indikatoren z.B. auch anhand von entsprechenden Intervallen für die Werte der jeweiligen Indikatoren definiert werden; auch ein Abstandsmaß in einem hochdimensionalen Vektorraum ist hierbei denkbar (eine Klasse wird dann bevorzugt über einen Basisvektor mit einzelnen Werten der jeweiligen Indikatoren als Vektoreinträgen definiert).

Andererseits umfasst jede Klasse 40 auch eine Zuordnung einer Mehrzahl von Parameterwerten 44 zu entsprechenden Signalverarbeitungsparametern 46 der Signalverarbeitungseinrichtung 12. Dies bedeutet: Die Signalverarbeitungsparameter 46, gemäß derer in der Signalverarbeitungseinrichtung 12 die beiden Eingangssignale 16, 17 zum Ausgangssignal 20 verarbeitet werden, werden in Abhängigkeit der gerade vorliegenden Klasse 40 für temporäre Umgebungssituationen (identifiziert anhand der akustischen und peripheren Indikatoren IndA, IndP) auf jeweilige Parameterwerte 44 eingestellt, welche für die vorliegende Klasse 40 definiert bzw. vorab festgelegt wurden.

Wird nun aber anhand der akustischen Indikatoren IndA und der peripheren Indikatoren IndP festgestellt (bspw. über ein Abstands- oder Ähnlichkeitsmaß, s.o.), dass die aktuell vorliegende temporäre Umgebungssituation 38 in keine der bekannten Klassen 40 fällt, so wird eine neue solche Klasse 40a definiert. Für die Definition werden dabei die aktuell vorliegenden Werte der akustischen und peripheren Indikatoren IndA, IndP verwendet. Die Definition kann dabei einerseits automatisch erfolgen. Andererseits kann über den Touchscreen 30 des Smartphones 25 auch eine erste Bestätigungsanfrage 48 an den Benutzer ausgegeben werden, ob dieser eine Definition einer neuen Klasse 40a für temporäre Umgebungssituationen wünscht, und die Definition bei der Bestätigung der Anfrage vorgenommen werden (z.B. kann der Benutzer die erste Bestätigungsanfrage 48 zurückweisen, wenn die aktuelle Situation mit hoher Sicherheit keine sich wiederholende temporäre Umgebungssituation darstellt).

Wird nun eine neue Klasse 40a für temporäre Umgebungssituationen definiert, so erfolgt dabei auch eine Zuweisung der entsprechenden Parameterwerte 44a für die zugehörigen Signalverarbeitungsparameter 46. Diese Zuweisung kann einerseits anhand einer akustischen Analyse der Umgebung (also mittels der Eingangssignale 16, 17) bzw. anhand einer ermittelten Hörsituation 42 erfolgen, wobei ggf. der Benutzer über den Touchscreen 30 eine Feinabstimmung vornehmen kann.

Insbesondere kann hierfür ein Testbetrieb der Signalverarbeitung mit vorläufigen Parameterwerten durchgeführt werden, und eine zweite Bestätigungsanfrage 49 an den Benutzer ausgegeben werden. Bei einer Bestätigung werden die vorläufigen Parameterwerte für die Definition der neuen Klasse 40a festgelegt, bei einer Zurückweisung kann der Benutzer einzelne Signalverarbeitungsparameter 46 anpassen, etwa durch sukzessive Abfragen durch das Hörsystem.

Die Parameterwerte 44a für die neue Klasse 40a können aber auch gewonnen werden, indem anhand der akustischen und peripheren Indikatoren IndA, IndP für die aktuell vorliegende temporäre Umgebungssituation 38 ein Ähnlichkeitswert 50 zu anderen Klassen 40 für temporäre Umgebungssituationen (bevorzugt jeweils zu einem Referenzvektor der betreffenden Klasse mit entsprechenden Indikatorwerten) gebildet wird. Anhand des Ähnlichkeitswertes 50 kann dann eine nächstähnliche Klasse 40b (oder auch eine zweit-ähnlichste Klasse etc.) zur vorliegenden temporäre Umgebungssituation 38 bestimmt werden. Die Parameterwerte 44a der neuen Klasse 40a können dann anhand der Parameterwerte 44 der nächst-ähnlichen Klasse 40b gewonnen werden. Auch hier kann der o.g. Testbetrieb mit vorläufigen Parameterwerten und der zweiten Bestätigungsanfrage 49 durchgeführt werden.

Insbesondere kann der Ähnlichkeitswert 50 dabei auch bereits für das Erkennen der neuen Klasse 40a verwendet werden. Bspw. kann dies dahingehend erfolgen, dass für die vorliegende temporäre Umgebungssituation 38 der Ähnlichkeitswert 50 bzgl. aller bekannter Klassen 40 ermittelt wird. Liegt der größte Ähnlichkeitswert 50 oberhalb eines vorgegebenen Grenzwertes (bei geeigneter Normierung ist dieser Grenzwert nahe 1 zu wählen), so fällt definitionsgemäß die vorliegende temporäre Umgebungssituation 38 in die bekannte Klasse 40. Erreicht jedoch für keine der bekannten Klassen 40 der Ähnlichkeitswert 50 mit der vorliegenden temporären Umgebungssituation 38 den Grenzwert, so wird entsprechend die neue Klasse 40a definiert, wobei für die Parameterwerte 44a die der nächst-ähnlichen Klasse 40b (die Klasse mit dem größten Ähnlichkeitswert 50) herangezogen werden können, und ggf. vom Benutzer geeignet modifiziert werden können.

Die Zuweisung der Parameterwerte 44a der neuen Klasse 40a kann auch dadurch erfolgen, dass die akustischen und peripheren Indikatoren IndA, IndP an ein Rechensystem 52 mit einer nicht näher dargestellten Datenbank übertragen werden, welches z.B. durch einen Cloud-Server gegeben sein kann. In besagter Datenbank sind eine Vielzahl von Klassendefinitionen 54 für temporäre Umgebungssituationen hinterlegt, wie sie bereits für andere Personen (insbes. durch diese selbst) durchgeführt wurden. Insbesondere sind dabei also für die einzelnen Klassendefinitionen 54, also für die für bzw. von anderen Personen vorgenommenen Definitionen von Klassen 40 für temporäre Umgebungssituationen, die jeweiligen Indikatoren IndA, IndP und die dazugehörigen Parameterwerte 44 hinterlegt. Anhand der hinterlegten Klassendefinitionen 54 kann von einer mit der Datenbank des Rechensystems 52 verbundenen Recheneinheit 56, welche als Teil des Rechensystems 52 ebenfalls auf der physischen Infrastruktur des Cloud-Servers implementiert sein kann, ein geeigneter Satz an Parameterwerten 44a für die neue Klasse 40a ermittelt und an das Hörsystem 1 zurück übertragen werden. Die Auswahl dieser geeigneten Parameterwerte 44a kann dabei insbesondere auch in ähnlicher Weise wie oben beschrieben anhand eines Ähnlichkeitsmaßes erfolgen, wobei die Ähnlichkeit der vorliegende temporären Umgebungssituation nun bzgl. der hiterlegten Klassendefinitionen überprüft wird.

Insbesondere können jedoch zu den einzelnen Klassendefinitionen 54 auch audiologische Daten (z.B. ein Audiogramm) und/oder biometrische Daten (z.B. Alter, Geschlecht, relevante Erkrankungen etc.) der jeweiligen Personen gespeichert sein, für welche die Klassendefinitionen 54 vorgenommen wurden. An die Datenbank des Rechensystems 52 sind dann für die Ermittlung der Parameterwerte 44a der neuen Klasse 40a auch entsprechende audiologische und/oder biometrische Daten des Benutzers des Hörinstruments 2 zu übertragen; diese können vorzugsweise im Smartphone 25 hinterlegt sein, mittels dessen die Übertragung der besagten Daten und grundsätzlich auch der akustischen und peripheren Indikatoren erfolgen kann.

Auch hier kann für die abschließende Bestimmung der Parameterwerte 44a für die Definition der neuen Klasse 40a zunächst der o.g. Testbetrieb mit vorläufigen Parameterwerten und der zweiten Bestätigungsanfrage 49 durchgeführt werden.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

### Bezugszeichenliste

- 1: Hörsystem
- 2: Hörinstrument
- 4: Hilfsgerät
- 6: Hörgerät
- 8: erster (elektroakustischer) Eingangswandler
- 9: erstes Mikrofon
- 10: zweiter (elektroakustischer)Eingangswandler
- 11: zweites Mikrofon
- 12: Signalverarbeitungseinrichtung
- 13: Lautsprecher
- 14: (elektroakustischer) Ausganswandler
- 15: Umgebungsschall
- 16: erstes Eingangssignal
- 17: zweites Eingangssignal
- 18: Ausgangssignal
- 20: Ausgangsschallsignal
- 21: erster Sensor
- 22: PPG-Sensor
- 23: weiterer Sensor
- 24: Beschleunigungssensor
- 25: Smartphone
- 26: Bluetooth-Verbindung
- 28: Prozessoreinheit
- 30: Touchscreen31 GPS-Sensor
- 32: kardiovaskuläre Kenngröße
- 34: körperliche Aktivität
- 36: Position
- 38: temporäre Umgebungssituation
- 40: Klasse für temporäre Umgebungssituationen
- 40a: neue Klasse (für temporäre Umgebungssituationen)
- 42: Hörsituation
- 44: Parameterwerte
- 44a: Parameterwerte (der neuen Klasse)
- 46: Signalverarbeitungsparameter
- 48: erste Bestätigungsanfrage
- 49: zweite Bestätigungsanfrage
- 50: Ähnlichkeitswert
- 52: Rechensystem
- 54: Klassendefinition
- 56: Recheneinheit

- IndA: akustische Indikatoren
- IndP: periphere Indikatoren

## Patentansprüche

1. Verfahren für ein Hörsystem (1) zum Anpassen einer Mehrzahl an Signalverarbeitungsparametern (46) eines Hörinstrumentes (2) des Hörsystems (1), gemäß denen wenigstens ein erstes Eingangssignal (16), welches durch einen ersten Eingangswandler (8) des Hörinstrumentes (2) aus einem Umgebungsschall (15) erzeugt wird, zu einem Ausgangssignal (18) verarbeitet wird, welches durch einen Ausgangswandler (14) des Hörinstrumentes (2) in ein Ausgangsschallsignal (20) umgewandelt wird,
- wobei anhand des ersten Eingangssignals (16), eine Anzahl an akustischen Indikatoren (IndA) für eine vorliegende temporäre Umgebungssituation (38) eines Benutzers des Hörinstrumentes (2) ermittelt wird,
- wobei anhand wenigstens eines ersten Sensors (21) des Hörsystems (1) eine Anzahl an peripheren Indikatoren (IndP) für die vorliegende temporäre Umgebungssituation (38) ermittelt wird,
- wobei anhand des bzw. der akustischen Indikatoren (IndA) und anhand des bzw. der peripheren Indikatoren (IndP) ermittelt wird, ob die vorliegende temporäre Umgebungssituation (38) in eine Klasse (40) aus einer Mehrzahl an bekannten Klassen (40) für temporäre Umgebungssituationen fällt, und
- wobei im Fall, dass die vorliegende temporäre Umgebungssituation (38) in keine der bekannten Klassen (40) für temporäre Umgebungssituationen fällt,
-- eine neue Klasse (40a) für temporäre Umgebungssituationen definiert wird, welche mittels des bzw. der jeweils ermittelten akustischen und peripheren Indikatoren (IndA, IndP) charakterisiert ist, und dabei
-- der Mehrzahl an Signalverarbeitungsparametern (46) jeweils eine entsprechende Mehrzahl an Parameterwerten (44a) zugewiesen wird, nach deren Maßgabe das erste Eingangssignal (16) bei einem späteren Vorliegen einer temporären Umgebungssituation (38) aus der neuen Klasse (40a) zu einem Ausgangssignal (18) zu verarbeiten ist.

2. Verfahren nach Anspruch 1,
wobei als akustische Indikatoren (IndA)
- eine Hörsituation (42), und/oder
- eine Kenngröße für einen Sprachanteil und/oder eine eigene Sprachaktivität des Benutzers im Umgebungsschall (15), und/oder
- eine Kenngröße für einen Rauschanteil im Umgebungsschall (15), und/oder
- eine Kenngröße für tonale Signale im Umgebungsschall (15) und/oder
- eine Kenngröße für eine Direktionalität im Umgebungsschall (15) ermittelt werden.

3. Verfahren nach Anspruch 1 oder Anspruch 2,
wobei als periphere Indikatoren (IndP)
- eine kardiovaskuläre Kenngröße (32), und/oder
- ein Bewegungszustand des Benutzers, und/oder
- eine Ortsposition (36) des Benutzers, und/oder
- eine Körpertemperatur des Benutzers, und/oder
- eine Kenngröße für ein Stressempfinden des Benutzers ermittelt werden.

4. Verfahren nach Anspruch 3,
wobei als erster Sensor zum Ermitteln des bzw. der peripheren Indikatoren
- ein Photoplethymographie-Sensor (22) und/oder
- ein Beschleunigungssensor (24) und/oder
- ein GPS-Empfänger (31), und/oder
- ein Thermometer und/oder
- ein Bioimpedanzsensor und/oder
- in Sensor einer elektrodermalen Aktivität und/oder
- ein Altimeter
verwendet werden.

5. Verfahren nach einem der vorhergehenden Ansprüche,
wobei für die Definition der neuen Klasse (40a) durch eine Bedieneinrichtung des Hörsystems (1) eine erste Bestätigungsanfrage (48) an den Benutzer gerichtet wird, und die Definition im Fall einer Bestätigung der Anfrage vorgenommen wird.

6. Verfahren nach einem der vorhergehenden Ansprüche,
wobei bei der Definition der neuen Klasse (40a) für die Zuweisung der Mehrzahl an Parameterwerten (44a) ein Ähnlichkeitswert (50) der neuen Klasse mit wenigstens einer bestehenden Klasse ermittelt wird, und die Mehrzahl an Parameterwerten (44a) für die neue Klasse anhand des Ähnlichkeitswertes (50) und anhand der Mehrzahl an Parameterwerten (44b) der wenigstens einen bestehenden Klasse (40b) zugewiesen wird.

7. Verfahren nach einem der vorhergehenden Ansprüche,
wobei bei der Definition der neuen Klasse (40a) für die Zuweisung der Mehrzahl an Parameterwerten (44a mittels einer Kommunikationseinrichtung des Hörsystems (1) der bzw. die ermittelten akustischen und peripheren Indikatoren (IndA, IndP) an ein vom Hörsystem (1) getrenntes Rechensystem (52) mit einer Datenbank übermittelt werden, in welcher eine Vielzahl an Klassendefinitionen (54) hinterlegt ist, und
wobei die Mehrzahl an Parameterwerten (44a) für die neue Klasse (40a) durch das Rechensystem (52) anhand des bzw. der ermittelten akustischen und peripheren Indikatoren (IndA, IndP) sowie anhand der Klassendefinitionen (54) zugewiesen wird.

8. Verfahren nach Anspruch 7,
wobei zusätzlich audiologische und/oder biometrische Daten des Benutzers an das Rechensystem (52) übermittelt werden,
wobei in der Datenbank weiter zur jeder Klassendefinition (54) zugehörige audiologische und/oder biometrische Daten einer der jeweiligen Klassendefinition (54) zugeordneten Person hinterlegt sind, und
wobei die Mehrzahl an Parameterwerten (44a) für die neue Klasse (40a) durch das Rechensystem (52) zusätzlich anhand der audiologischen und/oder biometrischen Daten des Benutzers sowie anhand der audiologischen und/oder biometrischen Daten der den jeweiligen Klassendefinitionen (54) zugeordneten Personen zugewiesen wird.

9. Verfahren nach einem der vorhergehenden Ansprüche,
wobei bei der Definition der neuen Klasse (40a) für die Zuweisung der Mehrzahl an Parameterwerten (44a) jeweils vorläufige Parameterwerte zugewiesen werden, und das erste Eingangssignal (16) nach Maßgabe der vorläufigen Parameterwerte für die Mehrzahl an Signalverarbeitungsparametern 46) zum Ausgangssignal (18) verarbeitet wird,
wobei das dem so erzeugten Ausgangssignal (18) entsprechende Ausgangsschallsignal (20) einem Gehör des Benutzers zugeführt wird, und
wobei eine zweite Bestätigungsanfrage (49) an den Benutzer gerichtet wird, und im Fall einer entsprechenden Bestätigung die vorläufigen Parameter als Mehrzahl an Parameterwerten (44a) für die Definition der neuen Klasse (40a) festgelegt wird.

10. Verfahren nach Anspruch 9,
wobei im Fall einer Ablehnung der zweiten Bestätigungsanfrage (49) eine Anfrage hinsichtlich einer Änderungen eines Parameterwertes (44) für einen bestimmten der Signalverarbeitungsparameter (46) an den Benutzer gerichtet wird, und wobei anhand einer entsprechenden Eingabe des Benutzers eine Änderung am besagten Parameterwert (44) für den bestimmten Signalverarbeitungsparameter (46) vorgenommen wird.

11. Hörsystem (1), umfassend
ein Hörinstrument (2) mit wenigstens einem Eingangswandler (8, 10), einer Signalverarbeitungseinrichtung (12) und einem Ausgangswandler (14),
- wobei der Eingangswandler (8, 10) dazu eingerichtet ist, aus einem Umgebungsschall (15) ein Eingangssignal (16, 17) zu erzeugen,
- wobei die Signalverarbeitungseinrichtung (12) dazu eingerichtet ist, das Eingangssignal (16, 17) nach Maßgabe einer Mehrzahl an Signalverarbeitungsparametern (46) zu einem Ausgangsignal (18) zu verarbeiten,
- wobei der Ausgangswandler (14) dazu eingerichtet ist, das Ausgangssignal (18) in ein Ausgangsschallsignal (20) umzuwandeln, und
einen ersten Sensor (21), welcher dazu eingerichtet ist, eine Anzahl an peripheren Indikatoren (IndP) für eine vorliegende temporäre Umgebungssituation (38) eines Benutzers des Hörinstruments (2) zu ermitteln,
wobei das Hörsystem (1) dazu eingerichtet ist, die besagte Mehrzahl an Signalverarbeitungsparametern (46) gemäß dem Verfahren nach einem der vorhergehenden Ansprüche anzupassen.
